# EUROPEAN PATENT APPLICATION

(11) **EP 4 837 540 A1**
(43) Date of publication of application: **11.11.2026**
(21) Application number: 25186470.8
(22) Date of filing: 30.06.2025
(51) Int. Cl.: A61F 2/28

(54) **INTEGRATED MAXILLOFACIAL IMPLANT FOR BONE STABILIZATION AND FACIAL AESTHETIC ENHANCEMENT**

(30) Priority: 08.05.2025 US 202519202199
(71) Applicant: Techfit Digital Surgery Inc., Daytona Beach, Florida 32114 (US)
(72) Inventor: TORO RESTREPO, Mauricio, Daytona Beach, Florida, 32114 (US); OCAMPO LÓPEZ, Valentina, Envigado, Antioquia (CO)
(74) Representative: Boult Wade Tennant LLP

(57) **Abstract**

An integrated maxillofacial implant for bone fixation and facial aesthetic correction is provided, which includes two zones, A and B, allowing the fixation of the lower and upper maxilla according to the fixation projections of each of these zones. This implant is manufactured from high-performance engineering polymers such as PEEK, PEKK, or carbon-fiber-reinforced PEEK, enhancing its mechanical properties and biocompatibility.

## Description

### Field of technology

The following belongs to the field of orthognathic surgery and specifically relates to an integrated maxillofacial implant designed for bone fixation and facial aesthetic enhancement, the implant includes two zones, A and B, allowing for the fixation of the upper and lower jaw according to the fixation projections of each zone. This implant is manufactured from high-performance engineering polymers such as PEEK, PEKK, or carbon-fiber-reinforced PEEK, enhancing its mechanical properties and biocompatibility.

### Background

Orthognathic surgery and facial implants are complementary procedures that address different aspects of facial aesthetics and functionality. Orthognathic surgery is a specialized surgical intervention that corrects dento-cranio-maxillofacial deformities by repositioning the maxilla and mandible to achieve facial balance in the patient. These deformities can lead to various issues, including sleep apnea, temporomandibular joint (TMJ) disorders, severe dental malocclusions, and skeletal disharmony.

After orthognathic surgery, facial implants may be required to correct any remaining aesthetic deficiencies or asymmetries. Facial implants are solid prostheses designed to modify the size of different facial areas, such as the chin, cheekbones, and jawline. They are used to enhance facial proportion and harmony, correct congenital deformities, and rehabilitate or replace facial structures disfigured by trauma, injuries, or accidents. Made from biocompatible materials such as silicone or polymethylmethacrylate (PMMA), these implants integrate well with facial tissues and improve appearance, although they are not designed to bear functional loads.

Currently, these procedures are performed in two separate surgical stages. However, recent advancements and integrated approaches enable both procedures to be carried out in a single intervention using advanced techniques such as 3D digital planning and customized surgical guides and plates. These methods allow for greater precision in the simultaneous placement of implants during orthognathic surgery. Nevertheless, combining both procedures in a single surgical session increases complexity and the risk of complications, such as infections, healing issues, or implant integration failures. As a result, the absence of a solution that simultaneously enables bone correction and aesthetic enhancement limits treatment efficiency and may impact the patient's quality of life during the rehabilitation period.

In the state of the art, there are embodiments of inventions such as the one disclosed in document US9066733B2, which describes an implant intended for use in orthognathic surgery of the maxilla. This implant may include a longitudinal plate member and a plurality of preformed fingers extending from an upper edge of the plate member. The longitudinal plate member is preformed to match the preoperative shape of the maxilla, and the fingers are preformed to conform to the shape of the maxilla. The fingers may be preformed to match either the preoperative or postoperative shape of the maxilla.

However, this document does not describe a facial implant for orthognathic surgery that simultaneously provides osteosynthesis support for bone alignment and stability while enhancing facial aesthetics. This is achieved by developing the implant according to the patient's anatomy based on anatomical and bone symmetry parameters. The implant is characterized by including direct fixation holes for bone attachment via screws, where a first portion of the implant is fixed to a first bone, and a second portion is fixed to a second bone. Additionally, the implant is manufactured from high-performance engineering polymers.

On the other hand, document CN116585074A refers to a combined design structure of a PEEK zygomatic prosthesis and its manufacturing method, relating to the technical field of zygomatic prostheses. Embodiments of the invention comprise a zygomatic prosthesis, which includes an I-shaped block and a grooved block. The I-shaped block is connected to the surface of the I-shaped block, and the surface of the grooved block is provided with an I-shaped groove that fits the I-shaped block. In this way, the I-shaped and grooved blocks are easily combined and inserted, facilitating the modular design of the zygomatic prosthesis and its placement in an appropriate position during prosthetic surgery. Embodiments of the invention overcome the disadvantage of the narrow space in the defective zygomatic area, which makes it difficult to properly place a complete prosthesis during surgery. Furthermore, one side of the I-shaped block, which is relatively distant from it, is connected to a zygomatic plate, allowing the zygomatic plate to be fixed in four directions through the combination of the zygomatic plate, the upper maxillary plate, and the zygomatic plates.

However, this document also presents the disadvantage of not specifically teaching a facial implant for orthognathic surgery in the cranio-maxillofacial region that integrates the function of an osteosynthesis plate by providing mechanical support for proper bone alignment and stability while also offering aesthetic benefits. The implant is developed according to the patient's anatomy based on anatomical and bone symmetry parameters. It is characterized by including direct fixation holes for bone attachment via screws, where a first portion of the implant is fixed to a first bone, and a second portion is fixed to a second bone. Additionally, the implant is manufactured from high-performance engineering polymers.

The document CN118141570A discloses a 3D-printed PEEK repair body designed to repair defects in the mandibular bone, belonging to the technical field of medical equipment. A customized PEEK repair body, which perfectly conforms to the bone shape around the mandibular defect, is designed using a 3D printing method based on the digital collection of patient data. The repair body is securely attached to the mandible using a titanium reconstruction plate. The elastic modulus of the prosthetic material is closer to that of human mandibular bone, which allows it to meet the body's normal physiological needs. Additionally, the material is translucent, non-magnetic, does not interfere with MRI imaging capabilities, does not generate metallic artifacts, and effectively prevents these artifacts from affecting the diagnosis of subsequent diseases and the design of corresponding clinical treatment plans.

However, this document has the drawback of not specifically disclosing a facial implant for orthognathic surgery in the cranio-maxillofacial region that integrates the function of an osteosynthesis plate. In embodiments, it does not describe an implant that provides mechanical support for proper bone alignment and stability while also offering aesthetic benefits. Furthermore, it does not disclose a design developed according to the patient's anatomy under parameters of anatomical and bone symmetry, nor does it describe an implant featuring direct fixation holes for securing to the bone with screws. Additionally, it does not specify a structure with two distinct portions allowing simultaneous fixation to both the upper and lower jaw, nor does it mention the use of high-performance engineering polymers for its fabrication.

The document GB2621847A discloses methods and systems for generating implants intended for use in aesthetic or reconstructive surgery. A three-dimensional (3D) model of a body portion of subject 102 is obtained. The model comprises: 3D photogrammetry data representing the subject's body portion and defining the desired external appearance of the portion; and a medical model representative of the body portion, which includes a first 3D structure, such as soft tissue, and a second 3D structure underlying the first, such as bone. An implant analysis 104 is conducted, which involves determining one or more volumes generated between the 3D photogrammetry data of the desired body portion and the second 3D structure, considering the structural properties of the first 3D structure. The implant generation 106 involves outputting data representative of the determined volumes to control the fabrication of the corresponding implants. Finite element analysis and spring-mass analysis are employed, involving structural meshes. Embodiments of the invention aim to improve the precision, efficiency, and predictability of the implant design and manufacturing process.

However, this document has the drawback of not specifically disclosing a facial implant for orthognathic surgery in the cranio-maxillofacial region that integrates the function of an osteosynthesis plate. In embodiments, it does not describe an implant that provides mechanical support for proper bone alignment and stability while also offering aesthetic benefits based on the patient's anatomy and bone symmetry. Furthermore, it does not disclose a design with direct fixation holes for securing to the bone with screws, nor does it specify a structure with a first and second portion to allow fixation to different bones. Additionally, the document does not mention the fabrication of the implant using high-performance engineering polymers.

The document US20200078156A1 mentions an alloplastic implant designed to cover gaps and irregularities along the osteotomy edge in the mandibular body. The alloplastic implant consists of an implant body made of biocompatible alloplastic material with a substantially "J"-shaped contour profile. The implant body features a vertical component with a substantially flat surface intended to be positioned against the frontal surface of the mandibular body, a lateral component with a semicircular profile that mimics the contour of the lower edge of the mandibular body, and a transverse component. The implant body is dimensioned to cover the gaps and irregularities along the mandibular body edge in a method for reconstructing such spaces, with the vertical component positioned to span a portion of the frontal surface of the mandibular body and the lateral component placed to overlap the lower edge of the mandibular body. Immobilization of the implant body relative to the mandibular body can be achieved through mechanical fixation.

However, this document has the drawback of not specifically disclosing a facial implant for orthognathic surgery in the cranio-maxillofacial region that integrates the function of an osteosynthesis plate. In embodiments, it does not describe an implant that provides mechanical support for proper bone alignment and stability while also offering aesthetic benefits based on the patient's anatomy and bone symmetry. Furthermore, it does not disclose a design featuring direct fixation holes for securing to the bone with screws, nor does it specify a structure with a first portion fixed to a first bone and a second portion fixed to a second bone. Additionally, the document does not mention the fabrication of the implant using high-performance engineering polymers.

### Summary

An aspect relates to an innovative facial implant specifically designed for bone fixation in orthognathic surgery in the cranio-maxillofacial region. This implant should not only serve the function of an osteosynthesis plate by providing essential mechanical support for proper bone alignment and stability but also offer significant aesthetic benefits. The implant must feature an advanced design that ensures effective integration with tissues, enhancing both the functionality and facial appearance of the patient.

### Brief description

Some of the embodiments will be described in detail, with references to the following Figures, wherein like designations denote like members, wherein:

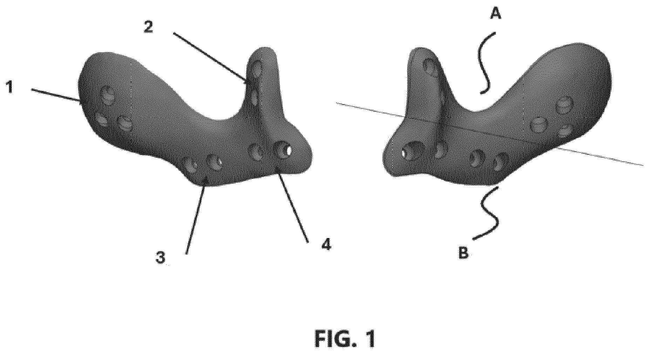

Figure 1 shows an isometric view of the maxillofacial implant of embodiments of the present invention, highlighting some of its components; and

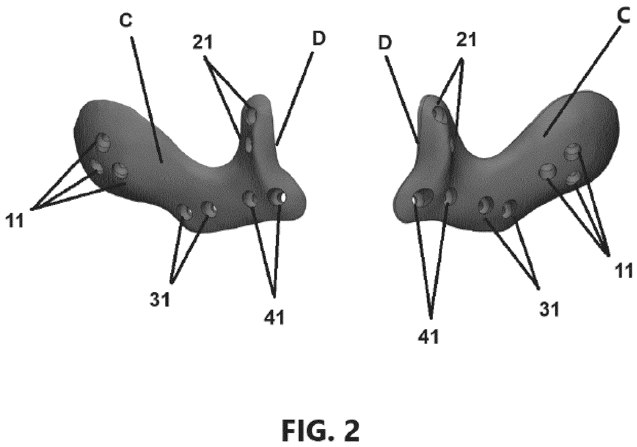

Figure 2 shows an isometric view of the maxillofacial implant of embodiments of the present invention, highlighting some of its components.

### Detailed description

Embodiments of the present invention relate to an integrated maxillofacial implant for bone fixation and facial aesthetic correction, characterized in that it comprises a first zone A and a second zone B, wherein the first zone A comprises a malar projection (1) and a paranasal projection (2), and the second zone B comprises a posterior alveolar space of the maxilla (3) and an anterior alveolar space of the maxilla (4).

The implant further comprises a front face C and a rear face D. The implant is manufactured as a single-piece, custom-made component using high-performance engineering polymers such as PEEK, PEKK, or PEEK, with or without continuous or discontinuous carbon fiber reinforcement.

The A and B zones of the implant are fixed to two different bone fragments; zone A is fixed to the upper maxilla, while zone B is fixed to the lower maxilla, thereby enabling the connection of two distinct bone fragments and bearing the loads associated with fixation in orthognathic surgery.

The malar projection (1) of the implant covers and provides projection to the malar region, while the paranasal projection (2) covers and provides projection to the paranasal region or the ascending process of the maxilla. Additionally, the posterior alveolar space (3) and anterior alveolar space (4) of the maxilla cover and provide projection to the lower part of the maxilla, thereby achieving an aesthetic effect and allowing the front face C of the implant to comprise an anatomical surface.

The implant includes angled through-holes for fixation, inclined between 15-30° to facilitate intraoral approach. The malar projection (1) region contains between 2 and 10 perforations (11), desirably 2. The paranasal projection (2) region contains between 2 and 10 perforations (21), desirably 2, avoiding perforation of the sinus and ensuring optimal bone quality. The posterior alveolar space (3) and anterior alveolar space (4) regions contain between 2 and 10 perforations (31), desirably 2, and between 2 and 10 perforations (41), desirably 2, respectively. The implant of embodiments of the present invention is secured using screws.

The implant features a geometry in the shape of two joined "L" structures. The first "L" is formed by the malar projection (1) and the posterior alveolar space of the maxilla (3), while the second "L" is formed by the paranasal projection (2) and the anterior alveolar space of the maxilla (4). The posterior face D of the implant is custom-fitted to the patient's specific anatomy.

In an embodiment, the implant of the present invention is symmetrical and is applied to both the left and right sides of the facial bones.

In an embodiment, the implant is manufactured using subtractive manufacturing techniques and is composed of high-performance engineering polymers. This allows for a customized anatomical adaptation for each patient, reducing the number of components in contact with tissue, thereby facilitating and accelerating the surgical procedure. Additionally, it ensures the necessary mechanical properties and flexibility for optimal functionality.

The key innovation of this implant lies in its adaptable thickness, allowing for precise anatomical adjustments tailored to each patient. This customization not only ensures effective bone fixation but also provides a unique opportunity to enhance projection in the malar region, contributing to a more harmonious facial contour and significantly improved aesthetic outcomes.

When embodiments of the present invention refers to fragments, they pertain to bone fragments that are joined through the implant described herein.

When embodiments of the present invention refers to projection, it denotes the augmentation of certain facial bones that require a specific geometry to ensure facial harmony and symmetry. For example, improving maxillofacial projection means achieving a more balanced and aesthetically pleasing facial contour.

The implant described provides the following benefits:
The implant is characterized by its division into two distinct zones:
- Zone A: Connects the upper maxilla.
- Zone B: Connects the lower maxilla.
This design enables a robust and efficient fixation between different bone structures.

The implant is designed to cover and provide projection to the following anatomical areas:
- Malar zone (anatomical area 1): This zone refers to the region of the malar bones or cheekbones, which form part of the lateral facial structure.
- Paranasal zone (anatomical area 2): Comprises the areas surrounding the nasal cavities, including the maxillary sinuses.
- Posterior alveolar zone of the maxilla (anatomical area 3): This zone includes the dental alveoli in the posterior part of the lower maxilla, where the roots of the molars and premolars are housed.
- Anterior alveolar zone of the maxilla (anatomical area 4): Refers to the dental alveoli in the anterior part of the lower maxilla, where the incisors and canines are located.

Thus, the main advantages of embodiments of the present invention are:
- Effective Bone Fixation: Provides solid mechanical support for proper bone alignment and stability during recovery, facilitating bone integration and consolidation.
- Aesthetic Enhancement: Enables customized anatomical adjustments that not only stabilize the bone but also improve projection in the maxillofacial region, contributing to a more harmonious and aesthetically pleasing facial contour.
- Reduction of Interventions: By integrating osteosynthesis functions and aesthetic improvements into a single implant, the need for additional procedures is reduced, simplifying the surgical plan and minimizing recovery time.
- Customized Design: Its precise manufacturing and advanced surgical planning ensure an exact anatomical fit, improving adaptability and reducing the risk of complications.
- Lower Risk of Infection: The reduction in the number of pieces and elements in contact with tissue decreases the risk of postoperative infections, improving procedural safety.
- Advanced Materials: Utilizes high-performance engineering polymers that offer superior mechanical properties, biocompatibility, lightweight characteristics, and flexibility, facilitating both tissue integration and surgical handling.
- Thickness Adaptability: Allows precise anatomical adjustments, improving bone fixation and providing the unique possibility of increasing projection in the malar region.

Although the present invention has been disclosed in the form of embodiments and variations thereon, it will be understood that numerous additional modifications and variations could be made thereto without departing from the scope of the invention.

For the sake of clarity, it is to be understood that the use of "a" or "an" throughout this application does not exclude a plurality, and "comprising" does not exclude other steps or elements.

## Claims

1. An integrated maxillofacial implant for bone fixation and facial aesthetic enhancement, comprising a first zone and a second zone, wherein the first zone A includes a malar projection and a paranasal projection, while the second zone B includes of a posterior alveolar space and an anterior alveolar space of the maxilla.

2. The maxillofacial implant according to claim 1, wherein it comprises a front face and a posterior face, wherein the implant is manufactured via machining as a single piece, customized to the patient's measurements, and made of high-performance engineering polymers such as PEEK, PEKK, or carbon-fiber-reinforced PEEK.

3. The maxillofacial implant according to claim 1, wherein zones A and B of the implant are fixed to two different areas; zone A is fixed to the upper maxilla, while zone B is fixed to the lower maxilla.

4. The maxillofacial implant according to claim 1, wherein the malar projection enhances the contour of the malar bone, while the paranasal projection provides structural support to the paranasal region and the ascending process of the maxilla, and wherein the posterior alveolar space of the maxilla and the anterior alveolar space of the maxilla improve projection in the lower maxillary region, with the front face of the implant forming an anatomical surface.

5. The maxillofacial implant according to claim 1, wherein it includes fixation holes with through-holes, angled at 15-30° for optimal screw placement, wherein a malar projection zone contains between 2 and 10 perforations, the paranasal projection zone contains between 2 and 10 perforations, and the posterior and anterior alveolar projection zones of the maxilla contain between 2 and 10 perforations and wherein the implant is secured to the bone using screws.

6. The maxillofacial implant according to claim 1, wherein a geometry including two joined "L" shapes: the first "L" formed by the malar projection and the posterior alveolar space of the maxilla, and the second "L" formed by the paranasal projection and the anterior alveolar space of the maxilla, and wherein the posterior face of the implant is custom-designed based on the patient's anatomical measurements.

7. The maxillofacial implant according to claim 1, wherein it is symmetrical and is applied to both the left and right sides of the facial bones.
